Europäisches Patentamt

European Patent Office

Office européen des brevets

(19)

(11) Publication number: **0 006 861**
**B1**

# EUROPEAN PATENT SPECIFICATION

(12)

(45) Date of publication of patent specification: **03.08.83**

(21) Application number: **78900025.4**

(22) Date of filing: **27.06.78**

(86) International application number:
**PCT/US78/00024**

(87) International publication number:
**WO 79/00014 11.01.79 Gazette 79/1**

(51) Int. Cl.³: **C 08 J 9/16, A 61 F 5/46,**
**B 01 D 47/16, B 32 B 5/18**

(54) **BIOLOGICALLY COMPATIBLE TAMPON SPONGE.**

(30) Priority: **27.06.77 US 810109**
**28.04.78 US 900864**

(43) Date of publication of application:
**23.01.80 Bulletin 80/2**

(45) Publication of the grant of the patent:
**03.08.83 Bulletin 83/31**

(84) Designated Contracting States:
**CH DE FR LU SE**

(56) References cited:
FR - A - 1 563 734
FR - A - 2 070 061
FR - A - 2 318 183
GB - A - 1 020 777
US - A - 2 687 729
US - A - 2 959 508
US - A - 3 075 930
US - A - 3 463 745
US - A - 3 632 361
US - A - 3 737 521
US - A - 3 762 414

(73) Proprietor: **VORHAUER LABORATORIES, INC.**
**130 McCormick Avenue**
**104, Costa Mesa, CA 92626 (US)**

(72) Inventor: **VORHAUER, Bruce, W.**
**50 Arboles**
**Irvine, CA 92715 (US)**
Inventor: **DOBBIE, Jr., Thomas, A.**
**2662 Vista Drive**
**Newport Beach, CA 92660 (US)**

(74) Representative: **Shipton, Gordon Owen et al,**
**W.P. THOMPSON & CO. Coopers Building Church**
**Street**
**Liverpool L1 3AB (GB)**

(56) References cited:
US - A - 3 806 350
US - A - 3 888 975
US - A - 3 903 232
US - A - 3 916 898
US - A - 3 918 452
US - A - 3 938 515
US - A - 3 978 855
US - A - 4 073 840
US - A - 4 092 387

Courier Press, Leamington Spa, England

## Biocompatible tampon contraceptive sponge

This invention relates to a biocompatible tampon contraceptive sponge and to a method of forming such a sponge.

Social scientists recognize that over population is one of the most serious problems which mankind must face and solve. As an example, in the United States the Department of Health, Education and Welfare released statistics recently which showed that 3.3 million babies were born in the United States in 1977, up 5% from the previous year. This marked an increase in the nation's birth rate for the first time since 1970. The problem is even more critical in underdeveloped countries.

In order to control our increasing world population, there exists a myriad of contraceptive devices to prevent unwanted pregnancy.

Each method attempts to achieve a contraceptive possessing the qualities of simplicity, acceptability, efficacy, non-toxicity and absence of adverse side effects. One class of such measures are mechanical contraceptives, such as the diaphragm, which are inserted into the vagina to completely occlude the orifice of the cervix, thus obstructing the migration of spermatozoa upward into the Fallopian tubes. Such contraceptives have serious application problems due to the wide variation in size and geometry of the vaginal canal and the cervical opening. These devices often require special insertion instruments and careful fitting, usually by a trained physician. Furthermore, since trained help in application is often necessary, these devices are ill-suited for the underdeveloped countries where they are needed most.

Chemical or spermicidal contraceptives are another well known method for attempting to prevent pregnancy. These devices consist of a carrier agent such as jellies and creams which provide partial obstruction of the cervix and in addition contain nontoxic chemical agents that immobilize sperm. The creams and jellies can be inserted high into the vagina with an applicator or can be encapsulated as capsules or suppositories. Capsule or suppositories are placed in the vagina shortly before coitus to allow sufficient time for them to melt and disperse their active ingredients. However, the use of these devices has resulted in unwanted pregnancies because the spermicidal material is not held for a sufficient length of time (which may be several hours) in the desired location in relation to the cervical opening. As a result of this inability to be effective alone, creams and jellies are often used in conjunction with other contraceptive devices. In addition, suppositories require skill in positioning the device in the depth of the vagina in order to prevent flow back out the vaginal opening.

Another type of mechanical device is the condom, a rubber device that surrounds the penis and contains the sperm after ejaculation. This widely used device suffers from an unnatural or desensitizing feeling to the male and female. In addition, the possibility of a perforation in the sheath and the problem of disposing of spent condoms, make the device less than the ultimate answer to unwanted pregnancy.

Recently, oral contraceptives have gained prominence. The "pill" is a female contraceptive which works by suppressing ovulation. One type of "pill" contains a combination of estrogen and progestogen. The Food and Drug Administration has recently published a brochure which reports that estrogen causes cancer in come animals, but studies were not confirmed that it causes cancer in humans. This brochure also states that the pill doubles a woman's chance of having a heart attack as well as increasing the risk of other circulatory problems.

Risk of heart attack is further increased if the woman is a smoker. Pill users who smoke are three times more likely to die of a heart attack than nonsmokers on the pill and ten times more likely than nonsmokers who do not use the pill.

The pill should not be taken by females who have certain ailments. In addition, undesirable side effects will sometimes occur when the female first takes the oral contraceptive. These include nausea and vomiting, increase in breast tenderness and engorgement, accentuation of acne, fluid retention, weight gain, increased vaginal discharge and breakthrough bleeding. Finally, there is documentation to the effect that the risk of death to the user may be on the order of 3 per 100,000.

Some pharmacological research has been focused recently upon producing male oral contraceptives. Alkylating agents and hormones to regulate spermatogenesis and thereby bring about a temporary or controlled sterility have been the subject of experimentation. However, alkylating agents are considered dangerous due to possible irreversible side effects and toxicity. Many unanswered questions remain with the use of hormones also.

In order to obviate some of the above deficiencies there have been attempts to combine mechanical and chemical contraceptive properties into a single device through the use of polymeric matrices carrying a pharmaceutically active amount of spermicide.

One such attempt is disclosed in U.S. Patent No. 3,261,353 by T. H. Johnson. Johnson teaches a device consisting of a laminate which contains a spermicide. The laminate consists of a nonporous rubber sheet cemented to a porous rubber or polymer foam. The spermicide is used in an amount ranging from 1 to 10% and is applied to the rubber or plastic foam through compression between rollers which may be enhanced by a surface application such as by painting or dipping.

This device possesses several deficiences however. For example, the need for a nonporous rubber sheet cemented to the polymer foam creates a device lacking the desired flexibility and natural feeling desired. Secondly, the foam contains a relatively low percentage of spermicide (1—10%). Thirdly, the spermicide would have a short retention time in the polymer foam.

A different approach to the problem is illustrated in U.S. Patent No. 3,514,517 issued to Furuse et al. Furuse teaches as one aspect of the invention the formation of a suppository from various surfactant/spermicides and polyethylene glycol. This process produces a suppository which melts and dissolves in the vaginal canal.

U.S. Patent No. 3,903,232 issued to Wood et al is directed to a foam structure which is prepared by using an isocyanate capped polyoxyethylene polyol reacted with large amounts of water. This specific polymer has been found to produce stable hydrophilic foams which are easily compressed, but which expand back to their original shape. Such foams contain a biocide, which upon contact with water is released. This enables such foams to be extremely useful for washing, wiping, cleaning the external body or used internally such as in the form of a tampon. However, it has been found that while the foam-like structure produced under the Wood et al method is extremely useful, such structure does not retain the biocide materials any great length of time and thus, the sponge has limited long term applicability.

The present invention is an improvement in the art of manufacturing sponges having a spermicide contained therein and which contain none of the shortcomings discussed hereinabove in connection with the prior art. The present invention enables a sponge to be formed which is flexible and which can be molded into a wide range of various shapes and sizes but which permits the retention of a spermicide therein for great lengths of time. Because of this feature, the sponge produced according to the present invention has distinct advantages in terms of an intravaginal device to be used by women as a birth control device. A sponge produced under the present invention could be used in the vagina for a period of up to one month, and yet still be biologically active in terms of the retention of a spermicide to improve the effectiveness of the device as a contraceptive.

Summary of the Invention

According to the present invention a method of forming a biocompatible tampon contraceptive sponge is provided characterized by the steps of: mixing spermicide-foaming agent with a water-catalyzed foam forming urethane prepolymer; and permitting said mixture of prepolymer and spermicide-foaming agent to foam and form a stable, flexible, sponge-like structure, the amount of the spermicide-foaming agent being greater than 30, and up to 50, dry weight percent based on the sponge.

The surprising synergistic result obtained from the polymerspermicide-foaming agent combination together with the unique method of manufacture and packaging produce a contraceptive device possessing all of the following properties:

(1) A simple device which can be inserted in privacy without a special applicator or counsel of a physician.

(2) A biocompatible, non-toxic, non-irritating device with no adverse side effects.

(3) A device which is so natural and soft in feeling that neither sexual partner is aware of its presence.

(4) A re-usable device with long lasting spermicidal activity, a factor of immense importance in underdeveloped countries.

(5) An effective vaginal contraceptive which need not be used in conjunction with creams, jellies or other suppositories.

(6) A device which is unitarily packaged *in situ* in a simple completely sanitary re-useable container.

The sponge possesses the mechanical properties of blocking the cervical opening when inserted and also of absorbing sperm through the porous cells. In addition, it possesses a chemical property by slowly releasing a spermicide.

The sponge is formed by mixing a polymeric material, or a monomer or prepolymer thereof, with an aqueous solution of the spermicide-foaming agent surfactant. After mixing, the blend is poured into a cavity polymer mold where it begins to foam into a sponge. The spermicide-foaming agent serves the critical function as a foaming agent which decreases the pore size of the resulting polymer foam. The resulting stable, flexible sponge is durable and has tear and fatigue strengths exceeding that of the prior art collagen sponges. Without the presence of the spermicide-foaming agent the physical characteristics of the sponge would drastically differ, probably the most important difference being that the pore size of the sponge would be too large to retain a spermicide for any appreciable period. As the foaming is near completion the mold is closed and sealed and the mold thus becomes package in which the device is commercially sold. This unique packaging procedure ensures a completely sanitary product.

A presently preferred embodiment of the invention is illustrated by way of example in the accompanying drawings. It is to be expressly understood, however, that the drawings are for the purpose of illustration and description only and are not intended as a definition of the limits of the invention.

Brief Description of the Drawings

Figure 1 is a partial cross-sectional view of a polymeric sponge which contains no collagen fibrils and less than 10% dry weight of the spermicide showing the foam-like structure.

Figure 2 is an artistic rendition of a magnified section of the polymeric sponge in Figure 1.

Figure 3 is a perspective view of the tampon sponge contraceptive.

Figure 4 is a cross-sectional view of a two cavity mold.

Figure 5 is a perspective view illustrating the placement of the loop and closing of the mold.

Figure 6 is a perspective view of the claimed invention as packaged.

Description of the Preferred Embodiment

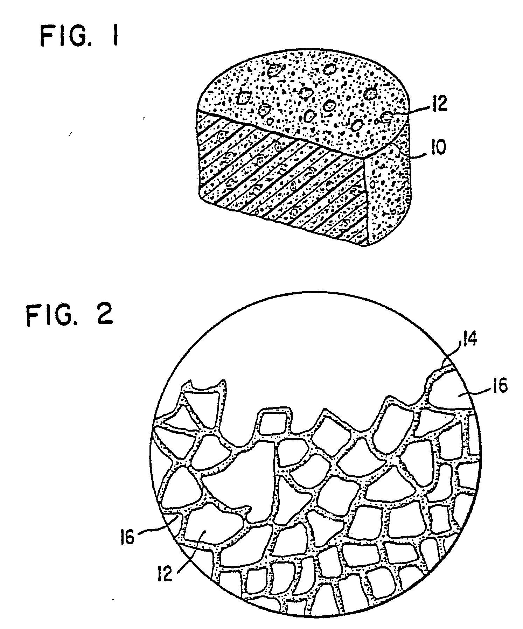

Referring first to Figures 1 and 2, one can see a prior art polymeric sponge 10 with the various pore-like structure 12 formed therein by the cross linking of the various polymeric chains 14. While a sponge made entirely of a polymeric foam without any spermicidal foaming agent is not within the scope of this invention, the type of stable polymeric foam which has been found to be particularly useful in this invention is the type of urethane foam described in U.S. Patent No. 3,903,232.

The interstitial spaces 16, as shown in Figure 2, formed by the cross linking of the various polymeric chains 14 are believed to provide the sponge which its unique flexing characteristic. However, it is believed that because the spaces 16 are relatively wide open, most types of pharmaceutically active materials in the sponge 10 would quickly be removed therefrom and thus, any prolonged biological activity would not remain. Accordingly, the use of pure urethane sponges as internal medical devices for supplying the user with a pharmaceutical agent has not been well received.

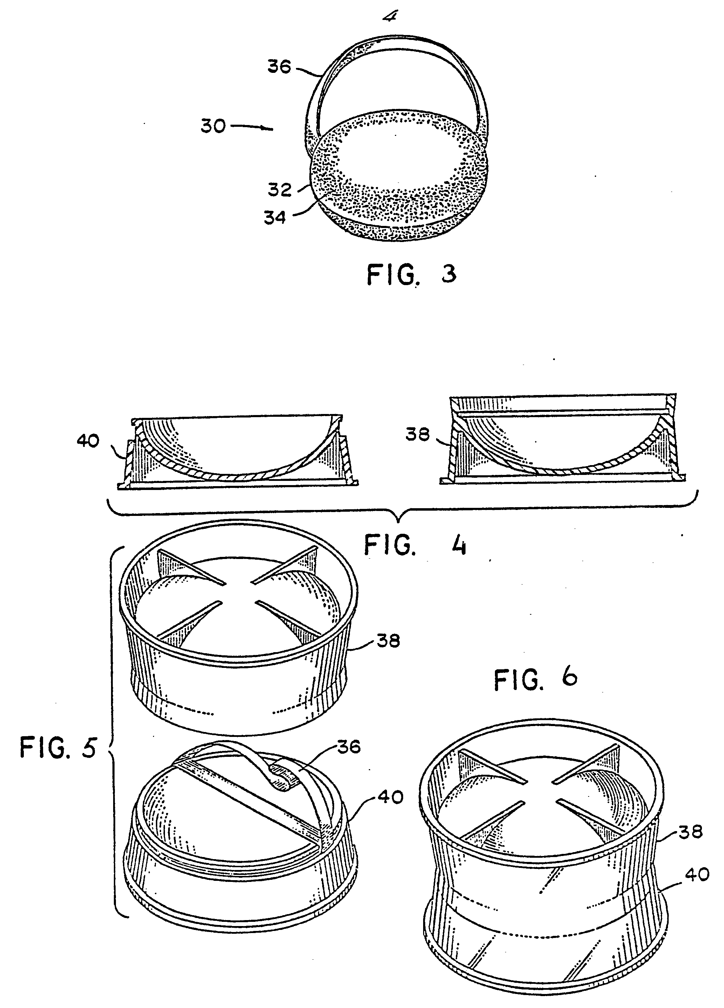

Figure 3 illustrates a tampon sponge 30. A polymeric sponge structure 32 is shown with tiny pores 34 which are in communication with the inner structure of the sponge (not shown). A flexible loop, 36, is attached to the structure 32 to provide a convenient removal means.

Polymeric sponge structure 32 is a soft pliable porous sponge in the shape of a flattened ball with a diameter of approximately two inches (5.08 cms). Preferably, the foam structure is prepared by using a polyurethane prepolymer reacted with large amounts of water. The preferred prepolymer is an isocyanate capped polyoxyethylene polyol.

The polyoxyethylene polyol used as a reactant in preparing the capped product to be foamed into the sponge of the present invention may have a weight average molecular weight of 200 to 1500, with a hydroxyl functionality of three or greater. The polyoxyethylene polyol is terminated or capped by reaction with a polyisocyanate. The reaction may be carried out in an inert moisture-free atmosphere, such as under a nitrogen blanket at atmospheric pressure at a temperature in the range of 0° to 120°C for a period of about twenty hours depending on the temperature and degree of agitation. The polyisocyanates used for capping the polyoxyethylene polyol include the various polyisocyanates set forth in the Wood et al disclosure.

Capping of the polyoxyethylene polyol may be effected using stoichiometric amounts of reactants. Desirably, however, an excess of isocyanate is used to ensure complete capping of the polyol. Thus, the ratio of isocyanate groups to the hydroxyl groups used for capping is between 1 to 4 isocyanate to hydroxyl molar ratio.

Water soluble or water dispersable biologically cross linking agents could be used in forming the prepolymer. Citric acid is one such possible cross linking agent which the present invention has incorporated albeit for other purposes than functioning as a cross linking agent, described later. However, the use of cross linking agents should be carefully considered inasmuch as the sponge of the present invention is to be used internally. Cross linking agents, such as the polyamines, i.e., triethylene amine or diaminotoluene are known irritants and therefore should be avoided.

The foaming and preparation of the contraceptive sponge structure will now be described.

A solution of water containing various additives including the spermicide/surfactant is mixed with a solution of the urethane prepolymer. The ratio of prepolymer to water is in the range of 30—150 parts by weight prepolymer to 100 parts by weight water. Conversely, the ratio of water to prepolymer is 66—133 parts by weight water to 100 parts by weight prepolymer. If either an excess or a deficiency of water is present, an incomplete reaction results. In the preferred embodiment, 77.77 parts of the prepolymer are mixed with 100 parts water in order that the volume ratios would result with two volumes of water to one volume of prepolymer. This 2:1 volume ratio, which is exemplary only, was chosen for convenience in order that in the manufacturing process two units of water could be added to one unit of prepolymer solution.

The final optimum ratios in parts by weight are: 4.5 parts water, 1.5 parts spermicide and 3.5 parts urethane prepolymer. This gives the above indicated volume ratio for the water to prepolymer of 2:1.

The aqueous solution contains the spermicide/surfactant.

One of the unexpected and surprising discoveries embodied in this invention is the synergistic combination of a spermicide foaming agent which also serves the function as a surfactant with a polyurethane prepolymer. One such spermicide/surfactant is nonylphenoxypoly (ethyleneoxy) ethanol, herein referred to as nonoxynol 9 which is a spermicide commonly used in vaginal birth control foam, gels and creams. Other examples of such spermicide surfactants which could be equivalent are p-menthanyl phenylpolyoxyethylene ether, nonyl phenylpolyoxynylethylene ether, octyl cresolpolyoxyethylene ether, polyoxyethylene oxypropylene stearate, polyoxyethylene laurate, glycerol ricinolate, di-iso-butyl phenylpolyoxyethylene ether, tri-isopropyl phenylpolyoxyethylene ether, mono-iso-octyl phenyl ether polyethylene glycol, methoxy polyoxyethylene glycol 500 laureate, polyoxyethylene stearylamine, benzalconium chloride, cetyl trimethylammonium bromide, methyl benzetonium chloride, benzetonium chloride, methyl dodecylxylylene-bis-trimethylammonium chloride, sodium dodecylsulfate, di-ethylhexyl sodium sulfosuccinate, nonylphenolpolyethylene sodium sulfate, sodium oleate, zinc phenolsulfonate, dodecylbenzene sulfonate or dodecyl diaminoethyl-glycine.

The high percentage of spermicide-foaming agent in the present invention is a highly surprising discovery and resulted from rigorous experimentation. The high percentage is unusual because heretofore it was well known that in producing polymer foams it is desirable to use as little surfactant as possible in order to maintain the purity of the resulting polymeric foam.

The percentage of spermicide foaming agent used is directly proportional to a decrease in the cellular pore size of the sponge.

Tables A and B illustrate the increasing ability of the sponge to retain spermicide after repeated washing with increasing percentages of the spermicide.

TABLE A

SPONGES FOR IN VITRO SPERMICIDAL TESTING

| | Urethane Only | Nonoxynol 10% | Nonoxynol 20% | Nonoxynol 30% | Preservatives | pH Adjusted w/Citric Acid |
|---|---|---|---|---|---|---|
| 1-Control | X | | | | | |
| 2-Test | | | | X | | |
| 3-Control | | | | | X | |
| 4-Test | | | | X | X | |
| 5-Control | | | | | X | X |
| 6-Test | | | | X | X | X |
| 7-Control | | | | | | X |
| 8-Test | | X | | | | |
| 9-Test | | | X | | | |

# TABLE B

SUMMARY OF SPERMICIDAL EFFECTIVENESS OF CONTRACEPTIVE

SPONGE ELUATES v. No. OF RINSES (Normal Saline, 20 ml Per Rinse)

| Sponge Number | | Rinse Number | | | | | | | | |
|---|---|---|---|---|---|---|---|---|---|---|
| | | 1 | 3 | 5 | 8 | 10 | 15 | 20 | 30 | 40 |
| 1 | Motility | 55% | | 55% | | 55% | 55% | 60% | 60% | 60% |
| | Quality | 3,2b,2a | | 3,2b,2a | | 3,2b | 3,2b | 3,2b | 3,2b | 3,2b |
| 2 | Motility | 0 | 0 | 0 | | 0 | 0 | 10% | 50% | 60% |
| | Quality | 0 | 0 | 0 | | 0 | 0 | 2a,1,3 | 3,2b | 3 |
| 3 | Motility | 55% | 55% | 55% | 55% | 55% | 55% | 55% | 55% | 55% |
| | Quality | 3 | 3 | 3 | 3 | 3 | 3 | 3 | 3 | 3 |
| 4 | Motility | 0 | | 0 | | 0 | 0 | 25% | 50% | 55% |
| | Quality | 0 | | 0 | | 0 | 0 | 3,2b | 3,2b | 3,2b |
| 5 | Motility | 55% | 55% | 55% | 55% | 55% | 55% | 55% | 55% | 55% |
| | Quality | 3,2b | 3,2b | 3,2b | 3,2b | 3,2b | 3,2b | 3,2b | 3,2b | 3,2b |
| 6 | Motility | 0 | | 0 | | 0 | 0 | 10% | 45% | 50% |
| | Quality | 0 | | 0 | | 0 | 0 | 2b,2a | 3,2b | 3 |

SUMMARY OF SPERMICIDAL EFFECTIVENESS OF CONTRACEPTIVE

SPONGE ELUATES v. No. OF RINSES (Normal Saline, 20 ml Per Rinse)

| Sponge Number | | Rinse Number | | | | | | | | |
|---|---|---|---|---|---|---|---|---|---|---|
| | | 1 | 3 | 5 | 8 | 10 | 15 | 20 | 30 | 40 |
| 7 | Motility | 50% | | 50% | | 50% | 55% | 55% | 60% | 60% |
| | Quality | 3,2b,2a | | 3,2b | | 3,2b | 3,2b | 3,2b | 3 | 3 |
| 8 | Motility | 0 | 20% | 40% | | 45% | 50% | 55% | 55% | 55% |
| | Quality | 0 | 3,2a | 3,2b | | 3,2b | 3,2b | 3,2b | 3 | 3 |
| 9 | Motility | 0 | 0 | 10% | | 20% | 35% | 55% | 55% | 55% |
| | Quality | 0 | 0 | 3,2b | | 3,2b | 3, 2b, 2a | 3 | 3 | 3 |

SPERM QUALITY CODE: Forward Progression — 3 — Normal
2b — Slow Motion

No Forward Progression — 2a — Vigorous Tail Motion
1 — Occasional Tail Beat
0 — Dead

The following conclusions can be obtained from the spermicidal effectiveness test:

(1) At least 30% of the nonoxynol 9 is required if the sponge is likely to be washed and re-used.

(2) There appears to be a sharp increase in spermicidal effectiveness and longevity between 20% nonoxynol 9 and 30% nonoxynol 9.

(3) A sponge containing 30% nonoxynol 9 retains spermicidal effectiveness through 15 rinses.

(4) Addition of preservative an a pH reducer (citric acid) alone or combined have little, if any, effect on spermicidal action.

(5) Addition of preservative increases spermicidal action slightly.

(6) Addition of a pH reducer (citric acid) increases spermicidal action slightly.

We have found that if 18 parts by weight of the urethan prepolymer are mixed with a solution consisting of 2 parts by weight of nonoxynol 9 surfactant/spermicide and 16 parts by weight of water this produces a sponge which has a nonoxynol dry weight content of 10% and which is soft and flexible but which does not retain the spermicide to a fully acceptable degree.

We have also found that if the percentage of spermicide-foaming agent rises above 50%, the sponge will become quite viscous and sticky which is of course an unacceptable condition. Thus, by increasing the dry weight percentages of spermicide foaming agent to an amount greater than 30% and up to 50%, preferably up to 40%, an extraordinary amount of agent is available in the sponge and the cellular pore size in the sponge is decreased sufficiently to produce a sponge with a great capacity to retain the agent. This decrease in cellular pore size in the sponge has made it unnecessary to include an additive such as collagen which is quite an important result since collagen presents several problems from a quality control standpoint, e.g. purification.

Several additives may optionally be added to the aqueous solution in addition to the spermicidal-foaming agent. For example, various preservatives, anti-fungicide, anti-bacterial agents and antioxidants may be added. Also, pH adjusters and buffers may be added. In the preferred embodiment a mixture of distilled water, and distilled, deionized demineralized water is mixed with 1/10 of 1% of benzoic acid, 2/10 of 1% of sorbic acid, 1/10 of 1% of sodium hydroxide, 2% of monhydrate citric acid and 5/100 of 1% of sodium metabisulphite.

The benzoic acid and sorbic act as preservatives to prevent bacterial or fungus growth from occuring especially when the sponge might be stored after use.

The citric acid is used to lower the pH of the solution to about 3.5. This is done to conform the sponge pH to the pH in the vaginal range which is approximately 4.0 to 5.0. If the vaginal pH is increased to much above that range, bacteria can begin to grow. For example, the gonococcus bacillus is very fragile and dies at low pH after only a short period of time. Another reason for dropping the pH to a more acidic level is that an acid environment is hostile to sperm and will decrease the sperm motility eventually to total incapacitation. The critic acid is then buffered with sodium hydroxide. The sodium hydroxide reacts with the citric acid to give a sodium dihydrogen citrate which provides the buffer action without reducing the citric acid content significantly. Another possible buffer which could be used is sodium citrate, but the sodium hydroxide is preferable.

The sodium metabisulphite serves as an antioxidant thereby depressing any oxidation processes which might occur.

This additive solution is given to disclose the preferred embodiment. Acceptable ranges for these additives can be found in the following publications: Remington Pharmaceutical Sciences, 15th Edition, 1975, published by Mack Publishing Company; the Martindale Pharmacopeia, published by the Pharmaceutical Press in London; the United States Pharmacoppeia, published by the United States Pharmacopoeia Convention, Inc.; and the Merck Index, published by Merck and Company.

Further insight into the effect of additives such as benzoic acid, sorbic acid and citric acid is obtained from Table C. Table C summarizes pH data for the nine sponges as listed in Table A.

## TABLE C

### SUMMARY OF pH DATA FOR CONTRACEPTIVE SPONGE ELUATES v. NUMBER OF RINSES

### (NORMAL SALINE, 20 ml PER RINSE)

| Sponge Number | Rinse Number | | | | | | | | |
|---|---|---|---|---|---|---|---|---|---|
| | 1 | 3 | 5 | 8 | 10 | 15 | 20 | 30 | 40 |
| 1 | 6.4 | 6.6 | 6.4 | 6.4 | 6.2 | 6.2 | 6.2 | 5.9 | 5.8 |
| 2 | 7.9 | 7.0 | 6.4 | 6.5 | 6.4 | 6.1 | 6.2 | 6.1 | 5.9 |
| 3 | 4.7 | 4.6 | 4.6 | 4.5 | 4.5 | 4.6 | 5.0 | 5.2 | 5.4 |
| 4 | 7.0 | 6.6 | 6.6 | 6.2 | 6.2 | 6.1 | 6.2 | 5.9 | 5.8 |
| 5 | 3.4 | 3.2 | 3.3 | 3.5 | 3.6 | 3.6 | 3.9 | 4.2 | 5.2 |
| 6 | 4.7 | 4.8 | 4.8 | 5.4 | 5.4 | 5.5 | 5.6 | 5.7 | 5.8 |
| 7 | 3.5 | 3.3 | 3.2 | 3.2 | 3.3 | 3.6 | 3.6 | 4.1 | 4.7 |
| 8 | 8.4 | 7.8 | 6.7 | 6.4 | 6.2 | 6.1 | 6.2 | 5.9 | 6.0 |
| 9 | 8.0 | 7.0 | 6.8 | 6.8 | 6.2 | 6.0 | 6.0 | 5.8 | 6.2 |

The conclusions that are obtained from the experiments depicted in Table C are as follows:

(1) Increasing percentages of nonoxynol 9 decreases the pH to more acidic levels.

(2) Preservatives (primarily benzoic acid and sorbic acid) contribute slightly to acid pH.

(3) Citric acid contributes strongly to an acidic pH level and 2% by dry weight brings the pH into that of the normal vaginal range (4 to 5).

(4) The effect of citric acid is essentially gone by the 20th rinse.

As stated earlier, two volumes of the water additive solution are mixed with one volume of the urethane prepolymer. This mixture is effected by a high shear mixer which produces a creaming action and the solution is ready to be poured or injected into a mold to begin its foaming action. The prepolymer can optionally be heated to temperatures of approximately 110 (43.3°C) to 120°F (48.9°C). These higher temperatures lower the cell density of the resulting sponge making the resulting sponge more porous and easier to handle.

It is critical in the type of product with which this invention is concerned that it be packaged in sanitary containers. This is often difficult where a product has to be handled after its manufacture. One method of solving the packaging and sanitization problem has been solved by molding the sponge in a polymer mold preferably having two cavities. Referring to Figure 4, two halves of the mold are shown as an upper half 38 and a lower half 40. The material and surface finish of the mold are important in several respects.

First, the surface of the mold is critical in forming the exterior characteristics of the sponge. For example, in forming the sponge upon foaming, there is a tendency for the sponge to form a shiny skinlike surface on its exterior. This is quite undesirable for the reasons that the sponge will not absorb the sperm as well nor will it release the spermicide through its outer pores as well. It has now been discovered that polypropylene or polyallomer are acceptable polymers in formation of the mold. The polyallomer which is a polymer manufactured by Eastman Chemical Products, Inc., Plastics Division, Kingsport, Tennessee, has been found to be especially advantageous particularly in view of the fact that it is generally recognized as safe as used in intravenous solution bottles. Both the polypropylene and polyallomer have been found to be successful in preventing any skin formation on the contraceptive sponge.

Secondly, and probably more important than the actual material of the mold, the surface finish of the mold must not be smooth. A smooth finish will produce a sponge with a shiny skin. However, a certain finish which is about 60 grit i.e., the finish resulting from use of a 60 grit sandblast, provides a rough finish which will prevent any skin formation.

The premixed urethane-water-spermicidal foaming agent solution is poured into the two halves of the mold. The foam forms by spontaneous exothermic reaction filling the mold cup. Just before the mold cups are filled, a soft dacron ribbon loop 36 as shown in Figure 5 is laid over one half of the mold 40 and the other half is quickly closed over half 40 with the loop inbetween. Dacron is a Registered Trade Mark.

With the mold closed, the polymer continues to foam with the air escaping out through a crack around the middle of the mold so that no air bubbles are trapped in the sponge. The shot size of material poured into the mold is measured precisely so that the sponge will just fill the container. The air escapes through the mold seam as the foam blows but the polymer solution will not escape since it is more viscous than the air. Shortly after the foaming operation is completed, perhaps 5 or 10 minutes later, a shrink wrap is then placed in an oven which shrinks that outer wrapping so that there is a tight seal and the product is now ready to be commercially sold, as shown in Figure 6. The mold therefore forms the final package and the product is thereby never removed from the mold. This produces a completely sanitary container.

The loop 36 on the sponge allows the tampon to be easily grasped for removal from the vaginal canal. The removal loop is made from 100% woven polyester which is a soft weave, non-irritating, and biocompatible. The loops are welded into circular shape with an ultrasonic weld.

## Claims

1. A method of forming a biocompatible tampon contraceptive sponge characterized by the steps of: mixing spermicide-foaming agent with a water-catalyzed foam forming urethane prepolymer; and permitting said mixture of prepolymer and spermicide-forming agent to foam and form a stable, flexible, sponge-like structure, the amount of the spermicide-foaming agent being greater than 30, and up to 50, dry weight percent based on the sponge.

2. A method as claimed in claim 1 characterized in that the urethane prepolymer is an isocyanate capped hydrophilic polyoxyethylene polyol.

3. A method as claimed in claim 1 or 2 characterized in that the spermicide-foaming agent is nonylphenoxypoly(ethylenoxy) ethanol.

4. A method as claimed in any one of the preceding claims wherein the dry weight percentage of spermicide-foaming agent is up to 40%.

5. A biocompatible tampon contraceptive sponge characterized by comprising: a spermicide-foaming agent in a dry weight percentage of greater than 30 and up to 50 dry weight percent based on the sponge and a water-catalyzed urethane polymer such that said urethane polymer forms a sponge substantially encapsulating said spermicide-foaming agent, said sponge having a plurality of pores, said spermicide-foaming agent acting to control the size of said pores such that said spermicide-foaming agent is slowly released from said sponge with use.

6. A sponge as claimed in claim 5 characterized in that the urethane polymer is water-catalyzed from an isocyanate capped hydrophilic polyoxyethylene polyol.

7. A sponge as claimed in claim 5 or 6 characterized in that the spermicide-foaming agent is nonylphenoxypoly(ethyleneoxy) ethanol.

8. A sanitary convenient packaged tampon sponge characterized in that it comprises a sponge as claimed in any of claims 5 to 7 formed in a mold, which mold when sealed, functions as the sanitary packaging for the sponge.

## Revendications

1. Procédé de formation d'un tampon absorbant contraceptif biocompatible, caractérisé par les étapes de: mélange d'un agent moussant spermicide avec un prépolymère d'uréthanne moussant catalysé par l'eau; et moussage dudit mélange de prépolymère et d'agent moussant spermicide avec formation d'une structure spongieuse flexible, stable, la quantité d'agent moussant spercicide étant supérieure à 30 et allant jusqu'à 50% en poids sur base sèche par rapport au tampon absorbant.

2. Procédé suivant la revendication 1, caractérisé en ce que le prépolymère d'uréthanne est un polyoxyéthylènepolyol hydrophile à groupe terminal isocyanate.

3. Procédé suivant la revendication 1 ou 2, caractérisé en ce que l'agent moussant spermicide est le nonylphénoxypoly(éthylèneoxy)éthanol.

4. Procédé suivant l'une quelconque des revendications précédentes, dans lequel le pourcentage en poids sur base sèche d'agent moussant spermicide atteint 40%.

5. Tampon absorbant contraceptif biocompatible, caractérise en ce qu'il comprend: un agent moussant spermicide en un pourcentage en poids sur base sèche de plus de 30 et allant jusqu'à 50, sur la base du tampon absorbant, et un polymère d'uréthanne catalysé par l'eau de manière que ledit polymère d'uréthanne forme une éponge enrobant pratiquement ledit agent moussant spermicide, ladite éponge présentant de nombreux pores, l'agent moussant spermicide agissant en limitant le diamètre des pores de manière qu'il soft lentement libéré de l'éponge au moment de l'utilisation.

6. Tampon absorbant suivant la revendication 5, caractérisé en ce que le polymère d'uréthanne est catalysé par l'eau à partir d'un polyoxyéthylènepolyol hydrophile à terminaison isocyante.

7. Tampon absorbant suivant la revendication 5 ou 6, caractérisé en ce que l'agent moussant spermicide est le nonylphénoxypoly(éthylèneoxy)éthanol.

8. Tampon absorbant sous emballage hygiénique pratique, caractérisé en ce qu'il comprend un tampon absorbant suivant l'une quelconque des revendications 5 à 7 mis en forme dans un moule qui, lorsqu'il est fermé, assume la fonction de l'emballage hygiénique pour le tampon absorbant.

## Patentansprüche

1. Verfahren zum Herstellen eines biokompatiblen empfängnisverhütenden Tamponschwamms, gekennzeichnet durch folgende Schritte: Mischen eines spermiziden Schäummittels mit einem wasserkatalysierten schaumbildenden Urethan-Prepolymer, sowie Schäumenlassen der Mischung aus Prepolymer und spermizidem Schäummittel und Bildung einer stabilen, flexiblen schwammartigen Struktur, wobei die Menge des spermiziden Schäummittels mehr als 30 und bis zu 50 Trockengewichts-Prozent bezogen auf den Schwam beträgt.

2. Verfahrens nach Anspruch 1, dadurch gekennzeichnet, daß das Urethan-Prepolymer ein mit einem Isozyanat abgedecktes hydrophiles Polyoxyäthylen-Polyol ist.

3. Verfahren nach Anspruch 1 oder 2, dadurch gekennzeichnet, daß das spermizide Schäummittel Nonylphenoxypoly-(Äthylénoxy-)Äthanol ist.

4. Verfahren nach einem der vorgehenden Ansprüche, dadurch gekennzeichnet, daß der Trockengewichtsanteil des spermiziden Schäummittels bis zu 40% beträgt.

5. Biokompatibler empfängnisverhütender Tamponschwamm, gekennzeichnet durch ein spermizides Schäummittel mit einem Trockengewichtsanteil von mehr als 30 und bis zu 50 Trockengewichts-Prozent bezogen auf den Schwamm und ein wasser-katalysiertes Urethan-Polymer derart, daß das Urethan-Polymer einen Schwamm bildet, der das spermizide Schäummittel im wesentlichen einkapselt, wobei der Schwamm eine Vielzahl von Poren aufweist und wobei das spermizide Schäum-mittel zur Steuerung der Größe der Poren dient, so daß das spermizide Schäummittel bei der Benutzung langsam aus dem Schwamm freigegeben wird.

6. Schwamm nach Anspruch 5, dadurch gekennzeichnet, daß das Urethan-Polymer aus einem mit Isozyanat abgedeckten hydrophilen Polyoxyäthylen-Polyol wasser-katalysiert ist.

7. Schwamm nach Anspruch 5 oder 6, dadurch gekennzeichnet, daß das spermizide Schäummittel Nonylphenoxypoly-(Äthylenoxy-)Äthanol ist.

8. Hygienisch verpackter Tamponschwamm, dadurch gekennzeichnet, daß er einen Schwamm aufweist, wie er in einem der Ansprüche 5 bis 7 beansprucht ist, der in einer Form gebildet ist, die im geschlossenen Zustand als hygienische Verpackung für den Schwamm dient.

FIG. 1

FIG. 2

0 006 861

FIG. 3

FIG. 4

FIG. 5

FIG. 6

2